# EUROPÄISCHE PATENTSCHRIFT

(11) **EP 2 739 386 B1**
(45) Veröffentlichungstag und Bekanntmachung des Hinweises auf die Patenterteilung: **10.06.2015**
(21) Anmeldenummer: 12740993.6
(22) Anmeldetag: 01.08.2012
(51) Int. Cl.: B01J 19/00, B01J 19/24, B01J 38/14, C07C 5/48, C07C 5/50

(54) **VERFAHREN ZUR DURCHFÜHRUNG VON KATALYTISCHEN AUTOTHERMEN GASPHASENDEHYDRIERUNGEN IN PRODUKTIONS- UND REGENERIERMODUS**
METHOD FOR CARRYING OUT CATALYTIC AUTOTHERMAL GAS PHASE DEHYDROGENATION IN PRODUCTION AND REGENERATION MODES
PROCÉDÉ DE DÉSHYDROGÉNATION CATALYTIQUE AUTOTHERMIQUE EN PHASE GAZEUSE FONCTIONNANT EN MODE DE PRODUCTION ET DE RÉGÉNÉRATION

(30) Priorität: 02.08.2011 EP 11176328
(43) Veröffentlichungstag der Anmeldung: 11.06.2014
(73) Patentinhaber: BASF SE, 67056 Ludwigshafen (DE)
(72) Erfinder: OLBERT, Gerhard, 69221 Dossenheim (DE); WEGERLE, Ulrike, 67550 Worms (DE); KOLIOS, Grigorios, 67435 Neustadt (DE); KOSTOVA, Albena, 68163 Mannheim (DE)
(86) Internationale Anmeldenummer: PCT/EP2012/064987
(87) Internationale Veröffentlichungsnummer: WO 2013/017608

(56) Entgegenhaltungen:
- WO-A1-2007/071654
- WO-A1-2011/067235
- WO-A1-2012/084609
- DE-A1- 10 246 119
- DE-A1-102007 006 647
- US-A- 4 560 815
- US-A- 5 405 814
- US-A1- 2004 030 214

## Beschreibung

Die Erfindung betrifft ein kontinuierliches Verfahren zur autothermen Gasphasendehydrierung eines kohlenwasserstoffhaltigen Gasstromes und Regeneration des Katalysators, der als Monolith ausgebildet ist, und eine Verwendung des Verfahrens.

Keramische oder metallische Monolithe sind als Katalysatorträger für Edelmetallkatalysatoren in der mobilen und stationären Abgasreinigung etabliert. Die Kanäle bieten der Strömung einen geringen Strömungswiderstand bei gleichzeitig gleichmäßiger Zugänglichkeit der äußeren Katalysatoroberfläche für gasförmige Reaktionsmedien. Dies ist vorteilhaft gegenüber regellosen Haufwerken, bei denen durch unzählige Umlenkungen bei der Strömung um die Partikel ein großer Druckverlust entsteht und die Katalysatoroberfläche eventuell nicht gleichmäßig genutzt wird. Der Einsatz von Monolithen ist generell interessant für katalytische Prozesse mit hohen Volumenströmen und adiabater Reaktionsführung bei hohen Temperaturen. Diese Merkmale treffen in der chemischen Produktionstechnik insbesondere für Dehydrierungsreaktionen zu, die in einem Temperaturbereich von 400 °C bis zu 700 °C ablaufen.

Fortschritte in der Katalysatortechnik ermöglichen die selektive Verbrennung des Dehydrierwasserstoffes in Anwesenheit von Kohlenwasserstoffen, wie beispielsweise in US 7,034,195 beschrieben. Eine derartige Fahrweise wird als autotherme Dehydrierung bezeichnet und erlaubt, Dehydrierreaktoren direkt zu beheizen, so dass aufwändige Vorrichtungen zur indirekten Vor- und Zwischenheizung des Reaktionsgemisches entfallen. Ein derartiges Verfahren ist beispielsweise in US 2008/0119673 beschrieben. Dieses Verfahren besitzt jedoch den gravierenden Nachteil, dass die Dehydrierung an einem heterogenen Katalysator in Pelletform durchgeführt wird: Der hohe Strömungswiderstand von Pelletschüttungen erfordert einen großen Reaktorquerschnitt und eine entsprechend niedrige Durchströmungsgeschwindigkeit, um den Druckabfall in der katalytisch aktiven Schicht zu begrenzen. Dieser Nachteil wird durch eine sehr aufwändige Vorrichtung zur Dosierung und Verteilung des Sauerstoffes ausgeglichen, was den Vorteil der autothermen Dehydrierung beeinträchtigt.

Aus der Patentameldung WO 2011/067235 ist ein Reaktor sowie ein Verfahren zur autothermen Gasphasendehydrierung von Kohlenwasserstoffen unter Einsatz von als Monolithe ausgebildeten heterogenen Katalysatoren bekannt, das eine Beherrschung der brennbaren Reaktionsmedien bei den hohen Reaktionstemperaturen, häufig im Bereich von etwa 400 bis 700 °C, sowie eine einfache Zugänglichkeit und Handhabung der Monolithe, insbesondere beim Rüsten des Reaktors sowie bei einem Katalysatorwechsel, gewährleistet.

WO 2011/067235 stellt einen Reaktor in Form eines liegenden Zylinders zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches an einem heterogenen Katalysator, der als Monolith ausgebildet ist, zur Verfügung, wobei
- der Innenraum des Reaktors durch ein lösbar, in Längsrichtung des Reaktors angeordnetes kreiszylindrisches oder prismatisches, in Umfangsrichtung gasdichtes, an beiden Stirnseiten desselben offenes Gehäuse G in
- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt ist,
- mit einer oder mehreren Zuführleitungen für den zu dehydrierenden kohlenwasserstoffhaltigen Gasstrom in den Außenbereich B, Umlenkung des zu dehydrierenden Kohlenwasserstoffstroms an einem Ende des Reaktors und Zuführung über einen Strömungsgleichrichter in den Innenbereich A,
- mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
- mit einer Abführleitung für das Reaktionsgemisch der autothermen Gasphasendehydrierung am Ende des Reaktors wie die Zuführleitung für den zu dehydrierenden Kohlenwasserstoffstrom.

An dem Reaktorende, an dem die Abführleitung für das Reaktionsgasgemisch der autothermen Gasphasendehydrierung angeordnet ist, ist vorteilhaft ein Rohrbündelwärmetauscher vorgesehen, mit einem Bündel von Rohren, durch die das Reaktionsgasgemisch zur autothermen Gasphasendehydrierung geleitet wird, sowie mit Zwischenräumen zwischen den Rohren, durch die, im Gegenstrom zum Reaktionsgemisch der autothermen Gasphasendehydrierung, der zu dehydrierende kohlenwasserstoffhaltige Gasstrom geleitet wird.

WO 2012/084609 beschreibt einen demgegenüber verbesserten Reaktor zur autothermen Gasphasendehydrierung, der sicherheitstechnische Vorteile aufweist und darüber hinaus die Problematik der Abdichtung des Rohrbündelwärmetauschers löst.

DE 10 2007 006647 beschreibt ein Verfahren zur Regenerierung eines im Rahmen einer heterogen katalysierten partiellen Dehydrierung eines Kohlenwasserstoffs desaktivierten Katalysatorsbett. Die bekannten Reaktoren zur autothermen Gasphasendehydrierung werden dergestalt gefahren, dass zwei gleichartige Reaktoren bereitgestellt werden, wobei ein erster Reaktor im Funktionsmodus der autothermen Gasphasendehydrierung betrieben wird, bis die Aktivität des Katalysators soweit nachlässt, dass dieser regeneriert werden muss, worauf der Reaktor in den Regeneriermodus umgeschaltet wird, und ein zweiter, gleichartiger Reaktor in den Produktionsmodus der autothermen Gasphasendehydrierung geschaltet wird.

In Anlagen zur autothermen Gasphasendehydrierung werden in der Regel sehr große Produktströme, häufig in einer Größenordnung von 150.000 bis 200.000 Jahrestonnen, erzeugt, die im Anschluss an die Dehydrierung weiteren Prozessstufen, d.h. insbesondere Aufarbeitungs- und/oder Reaktionsstufen, zugeführt werden. Diese Prozessstufen müssen kontinuierlich laufen, da bei den großen Massenströmen ein Neustart oder auch ein Lastwechsel zu aufwändig wären.

Darüber hinaus ist bei der Fahrweise nach dem Stand der Technik mit zwei Reaktoren, die alternierend im Produktions- bzw. Regeneriermodus betrieben werden, bei großtechnischen Verfahren der Aufwand für das Umschalten zwischen den beiden Fahrweisen bezüglich Investitionskosten, Sicherheit, Arbeitszeit, etc. hoch. Ein Scaleup ist aufwändig, weil für eine Kapazitätserhöhung gleichzeitig zwei Reaktoren entsprechend größer ausgelegt werden müssen. Auch ist bereits in der Regel bei der Fahrweise nach dem Stand der Technik mit zwei im alternierenden Produktions- bzw. Regeneriermodus betriebenen Reaktoren ein Pufferbehälter erforderlich, um die Umschaltzeit zu kompensieren.

Es war daher Aufgabe der Erfindung, ein kontinuierliches Verfahren zur autothermen Gasphasendehydrierung zur Verfügung zu stellen, das die obigen Nachteile nicht aufweist.

Die Aufgabe wird durch ein Verfahren gemäss Anspruch 1 gelöst.

Mit zunehmender Betriebsdauer der autothermen Gasphasendehydrierung nimmt die Aktivität des Dehydrierkatalysators ab, mit der Folge, dass das Reaktionsgasgemisch beim Austritt aus dem Reaktor heißer wird.

Die Erfindung nutzt als Signal für die Umschaltung vom Produktionsmodus in den Regeneriermodus diese Temperaturerhöhung des Reaktionsgasgemisches beim Austritt aus der letzten katalytisch aktiven Zone und vor dem Eintritt in den Wärmetauscher: hierzu wird die Temperatur desselben kontinuierlich, in dem Fachmann bekannter Weise, z.B. mittels eines Thermoelementes, gemessen und mit dem Messwert für die Temperatur zum Zeitpunkt Null verglichen. Hierbei wird der Zeitpunkt Null wie folgt festgelegt: beim Starten des Produktionsmodus schwankt die Temperatur zunächst, wie üblich, während einer Startphase, bis sich ein quasistationärer Zustand einstellt, ab dem der zeitliche Temperaturgradient linear im Bereich von etwa 0 bis 2 Kelvin pro Stunde ansteigt. Dieser Zeitpunkt, ab dem sich der quasistationäre Betriebszustand mit weitgehend linearem zeitlichen Anstieg des Temperaturgradienten einstellt, entspricht dem Zeitpunkt, ab dem der Umsatz der autothermen Gasphasendehydrierung nahezu konstant ist, d.h. vorliegend insbesondere um nicht mehr als 1 %, bezogen auf den Endumsatz, schwankt. Dieser Zeitpunkt, ab dem der Umsatz der autothermen Gasphasendehydrierung um nicht mehr als 1 %, bezogen auf den Endumsatz, schwankt, wird vorliegend als Zeitpunkt Null für die Messung der Temperaturerhöhung des Reaktionsgasgemisches beim Austritt aus der letzten katalytisch aktiven Zone festgelegt.

In einer bevorzugten Ausführungsform wird als Zeitpunkt Null für die Messung der Temperaturerhöhung des Reaktionsgasgemisches beim Austritt aus der letzten katalytisch aktiven Zone und vor dem Eintritt in den Wärmetauscher der Zeitpunkt festgelegt, zu dem der Umsatz um nicht mehr als 0,5 %, bezogen auf den Endumsatz, schwankt.

Weiter bevorzugt wird als Zeitpunkt Null für die Messung der Temperaturerhöhung des Reaktionsgasgemisches beim Austritt aus der letzten katalytisch aktiven Zone und vor dem Eintritt in den Wärmetauscher der Zeitpunkt festgelegt, zu dem der Umsatz um nicht mehr als 0,2 %, bezogen auf den Endumsatz, schwankt.

Es ist dem Fachmann allgemein bekannt, wie er kontinuierlich den Umsatz einer autothermen Gasphasendehydrierung ermittelt. Der Umsatz bezeichnet in der chemischen Reaktionstechnik bekannterweise den Anteil eines Ausgangsstoffes, der beim Verlassen des Reaktors in andere chemische Stoffe durch chemische Reaktion umgewandelt wurde (vergleiche Wikipedia). Dieser Anteil lässt sich durch bekannte analytische Verfahren in dem Fachmann bekannter Weise kontinuierlich ermitteln, insbesondere durch Online-Gaschromatographie (GC) oder Fourier-Transform-Infrarotspektroskopie (FTIR). Die Antwortzeit der GC ist bekannterweise im Bereich von 20 Minuten bzw. im Bereich von etwa 1 Minute im Falle der Mikro-GC. Die FTIR hat eine Antwortzeit im Bereich von 30 Sekunden.

In einer bevorzugten Ausführungsform kann die relativ aufwändige Online-Analytik zur Umsatzbestimmung durch eine einfache Temperaturmessung ersetzt werden:
Es wurde gefunden, dass der Zeitpunkt Null für die Messung der Temperaturerhöhung des Reaktionsgasgemisches, d.h. der Zeitpunkt, ab dem der Umsatz der autothermen Gasphasendehydrierung um nicht mehr als 1 %, bezogen auf den Endumsatz, schwankt, bei Einsatz einer einzigen katalytisch aktiven Zone im Innenbereich A dem Zeitpunkt entspricht, ab dem die Temperatur des Reaktionsgasgemisches über einen Zeitraum von mindestens 15 Minuten linear ansteigt. Der Zeitpunkt Null kann somit durch eine einfache Temperaturmessung des Reaktionsgasgemisches bestimmt werden. Dabei kann in der bevorzugten Ausführungsform auf die aufwändigere Online-Analytik zur Umsatzbestimmung verzichtet werden; darüber hinaus ist auch die Dynamik der Temperaturmessung schneller als die Dynamik von Konzentrationsmessungen.

Für die Ausführungsform, wonach zwei oder mehrere hintereinander angeordnete katalytisch aktive Zonen vorgesehen sind, wird der Zeitpunkt Null für die Messung der Temperaturerhöhung des Reaktionsgasgemisches beim Austritt aus der letzten katalytisch aktiven Zone als der Zeitpunkt bestimmt, ab dem die Temperatur beim Austritt aus jeder katalytisch aktiven Zone, jeweils gegenüber der Temperatur beim Austritt aus der unmittelbar vorhergehenden katalytisch aktiven Zone, stärker ansteigt.

Der Produktionsmodus wird gefahren, solange die Temperaturerhöhung des Reaktionsgasgemisches beim Austritt aus der letzten katalytisch aktiven Zone und vor dem Eintritt in den Wärmetauscher gegenüber dem vorstehend definierten Startzeitpunkt (Zeitpunkt Null) den oben angegebenen Wert von 5 K nicht überschreitet. D i es bedeutet, dass zu jedem Zeitpunkt, solange die Temperaturerhöhung des Reaktionsgasgemisches in der oben definierten Weise den oben definierten Wert nicht überschreitet, vom Produktionsmodus in den Regeneriermodus umgeschaltet werden kann. Aus wirtschaftlichen Gründen ist es bevorzugt, die maximale Dauer für den Produktionsmodus auszunützen, d.h. möglichst nahe an die obige Grenze für die Temperaturerhöhung zu fahren.

In einer vorteilhaften Ausführungsform wird der Produktionsmodus beendet und der Reaktor in den Regeneriermodus umgeschaltet, sobald die Temperaturerhöhung des Reaktionsgasgemisches beim Austritt aus der letzten katalytisch aktiven Zone und vor dem Eintritt in den Wärmetauscher zu dem wie vorstehend definierten Startzeitpunkt 4 K überschreitet.

Weiter vorteilhaft wird der Produktionsmodus beendet und der Reaktor in den Regeneriermodus umgeschaltet, sobald die Temperaturerhöhung des Reaktionsgasgemischs beim Austritt aus der Abführleitung dem wie vorstehend definierten Startzeitpunkt 3 K überschreitet.

Wie vorstehend bereits definiert, wird als Startzeitpunkt (Zeitpunkt Null) der Zeitpunkt festgelegt, ab dem der Umsatz der autothermen Gasphasendehydrierung um nicht mehr als 1 %, bezogen auf den Endumsatz, schwankt, bevorzugt der Zeitpunkt, zu dem der Umsatz um nicht mehr als 0,5 %, bezogen auf den Endumsatz, schwankt, weiter bevorzugt der Zeitpunkt, zu dem der Umsatz um nicht mehr als 0,2 %, bezogen auf den Endumsatz, schwankt.

Im erfindungsgemäßen Verfahren wird die Betriebszeit im Produktionsmodus wie oben definiert begrenzt; anschließend wird in den Regeneriermodus unter Einsatz eines Regeneriergases umgeschaltet, das einen hohen Anteil von mindestens 10 Gew.-% Sauerstoff, bezogen auf das Gesamtgewicht desselben, enthält, und das es ermöglicht, innerhalb einer relativ kurzen Regenerierzeit den Reaktor wieder für den Produktionsmodus zur Verfügung zu stellen.

Durch die erfindungsgemäße Betriebsweise ist es möglich, dass in jedem Betriebszyklus, umfassend jeweils einen Produktionsmodus und einen Regeneriermodus, maximal 15 % der gesamten Betriebszeit auf den Regeneriermodus entfällt.

Die Betriebszeit für einen Produktionsmodus ist typischerweise 3 Stunden oder länger.

Bevorzugt entfällt in jedem Betriebszyklus, umfassend jeweils einen Produktionsmodus und einen Regeneriermodus, maximal 10 %, weiter bevorzugt maximal 5 % der gesamten Betriebszeit auf den Regeneriermodus.

Das erfindungsgemäße Verfahren wird in einem Reaktor durchgeführt, wie er aus WO 2012/084609 bekannt ist. Hierbei handelt es sich um einen Reaktor in Form eines Zylinders oder Prismas, wobei der Innenraum des Reaktors durch ein in Längsrichtung des Reaktors angeordnetes kreiszylindrisches oder prismatisches gasdichtes Gehäuse G in
- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt ist, und wobei
- an einem Reaktorende im Anschluss an das Gehäuse G ein Wärmetauscher vorgesehen ist
- mit einer oder mehreren Zuführleitungen für den zu dehydrierenden kohlenwasserstoffhaltigen Gasstrom,
- mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
- mit einer Abführleitung für das Reaktionsgasgemisch der autothermen Gasphasendehydrierung, wobei
- der Außenbereich B mit einem unter den Reaktionsbedingungen der autothermen Gasphasendehydrierung inerten Gas beaufschlagt ist und
- der zu dehydrierende kohlenwasserstoffhaltige Gasstrom über eine Zuführleitung in den Wärmetauscher eingeleitet, im Wärmetauscher durch das Reaktionsgasgemisch im Gegenstrom durch indirekten Wärmetausch aufgeheizt und weiter an das dem Wärmetauscher entgegengesetzte Ende des Reaktors geleitet, dort umgelenkt, über einen Strömungsgleichrichter in den Innenbereich A eingeleitet, und in den Mischzonen mit dem Sauerstoff enthaltenden Gasstrom vermischt wird, worauf im Innenbereich A des Reaktors die autotherme Gasphasendehydrierung stattfindet.

Der Reaktor ist bevorzugt als liegender Zylinder oder Prisma ausgebildet.

Der Reaktor ist mit einem Reaktormantel, d.h. einer drucktragenden Hülle, ausgestattet, die nicht mediumberührt ist, weder durch den kohlenwasserstoffhaltigen noch durch den sauerstoffhaltigen Strom.

In Längsrichtung des Reaktors ist ein zylindrisches oder prismatisches Gehäuse G angeordnet, das den Innenraum des Reaktors in einen Innenbereich A und einen konzentrisch um denselben angeordneten Außenbereich B aufteilt.

Der Außenbereich B ist mit einem unter den Reaktionsbedingungen der autothermen Gasphasendehydrierung inerten Gas beaufschlagt, d.h. einem Gas oder Gasgemisch, das an der Reaktion der autothermen Gasphasendehydrierung nicht unmittelbar beteiligt ist, insbesondere ausgewählt aus Wasser, Kohlendioxid, Stickstoff und Edelgasen oder Gemischen hiervon. Bevorzugt wird als unter den Reaktionsbedingungen der autothermen Gasphasendehydrierung inertes Gas Wasserdampf eingesetzt, da dieser in einfacher Weise, durch Auskondensieren, aus dem Reaktionsgasgemisch wieder abgetrennt werden kann.

Bevorzugt wird das unter den Reaktionsbedingungen der autothermen Gasphasendehydrierung inerte Gas als Purge-Gasstrom mit einem geringen Massenstrom im Vergleich zum Massenstrom des kohlenwasserstoffhaltigen Gasstromes, d.h. einem Massenstrom von 1/5 bis 1/100, bevorzugt einem Massenstrom von 1/10 bis 1/50, bezogen auf den Massenstrom des kohlenwasserstoffhaltigen Gasstromes, unter geringem Überdruck von 2 bis 50 mbar, bevorzugt von 25 bis 30 mbar, bezogen auf den Druck im Innenbereich A durch denselben geleitet.

Der Purge-Gasstrom kann vorteilhaft durch den Außenbereich B geleitet werden, indem er an einem Reaktorende über eine oder mehrere Zuführleitungen in den Außenbereich B des Reaktors eingeleitet und am entgegengesetzten Reaktorende in den Innenbereich A des Reaktors weitergeleitet wird, bevorzugt über eine oder mehrere, vorteilhaft in einem Winkel verschieden von 90° zur Zuführleitung, für den zu dehydrierenden kohlenwasserstoffhaltigen Gasstrom angeordnete Verbindungsleitung(en).

Die eine oder mehreren Verbindungsleitung(en), die den Purge-Gasstrom aus dem Außenbereich B in den Innenbereich A leiten, sind bevorzugt rückströmungsfrei ausgebildet, beispielsweise durch eine gewendelte Form derselben. Der Einlass aus dem Außenbereich B in die Verbindungsleitung für den Purge-Gasstrom, soll bevorzugt so hoch wie möglich im Außenbereich B des Reaktors angeordnet sein.

Der Purge-Gasstrom spült permanent den Außenbereich B des Reaktors und hält diesen frei von Komponenten des Reaktionsgasgemischs.

An einem Ende des Gehäuses G ist ein Wärmetauscher angeschlossen, der insbesondere ein Rohrbündelwärmetauscher oder ein Plattenwärmetauscher sein kann. Für den Fall des Rohrbündelwärmetauschers ist der Anschluss zwischen demselben und dem Gehäuse G so ausgebildet, dass der Innenbereich A mit dem Innenraum der Rohre des Rohrbündelwärmetauschers kommuniziert. Für den Fall eines Plattenwärmetauschers kommuniziert der Innenbereich A des Reaktors mit den Spalten zwischen den Platten des Plattenwärmetauschers.

Der Zwischenraum zwischen den Rohren des Rohrbündelwärmetauschers bzw. zwischen jeweils zwei zu einer Wärmetauscherplatte zusammengeschweißten Blechen des Plattenwärmetauschers ist über eine Leitung, die an das dem Wärmetauscher entgegengesetzte Ende des Reaktors führt und dort umgelenkt wird, mit dem dem Wärmetauscher entgegengesetzten Ende des Gehäuses G und somit dem Innenbereich des Reaktors gasdicht gegenüber dem Außenbereich B verbunden.

Der kohlenwasserstoffhaltige Strom wird durch den Zwischenraum zwischen den Rohren des Rohrbündelwärmetauschers bzw., für den Fall eines Plattenwärmetauschers, durch die Zwischenräume zwischen den jeweils eine Wärmetauscherplatte bildenden Blechen geleitet, mit dem im Gegenstrom durch die Rohre bzw. durch die Spalte zwischen den Platten des Plattenwärmetauschers zirkulierenden Produktgasstrom aufgeheizt, an das entgegengesetzte Ende des Reaktors geführt, dort umgelenkt und in den Innenbereich A des Gehäuses eingeleitet.

Die autotherme Gasphasendehydrierung findet an einem heterogenen Katalysator statt, der in Form von Monolithen vorliegt.

Die nebeneinander, übereinander und hintereinander zu einer Packung gestapelten Monolithe sind bevorzugt in einer Blähmatte oder in einem Mineralfaservlies eingehüllt und in eine Einhausung mit Verspanneinrichtung eingesetzt. Als Mineralfaservliese werden bevorzugt Vliese eingesetzt, wie sie für den Einsatz für Abgaskatalysatoren bekannt sind, beispielsweise Interam^{®} Lagermatten der Firma 3M^{®}.

Die in Blähmatten eingehüllten Monolithe sind in einem Gehäuse angeordnet, das bevorzugt thermisch isoliert, weiter bevorzugt lose im Reaktor gelagert und weiter bevorzugt als Quader ausgebildet ist.

Bevorzugt sind die Seitenwände des als Quader ausgebildeten Gehäuses einzeln abnehmbar ausgebildet, dergestalt, dass eine komplette Packung oder einzelne Monolithe einer Packung aus einer katalytisch aktiven Zone ausgetauscht werden können.

Die einzelnen Monolithe werden nebeneinander, übereinander und hintereinander, in der erforderlichen Anzahl, um eine katalytisch aktive Zone auszufüllen, unter Ausbildung einer Packung, gestapelt.

Vor jeder Packung ist jeweils eine Mischzone mit festen Einbauten, die nicht katalytisch aktiv sind, vorgesehen. In der Mischzone erfolgt die Vermischung des kohlenwasserstoffhaltigen Gasstromes mit dem Sauerstoff enthaltenden Strom, wobei in der in Strömungsrichtung zuerst angeströmten Mischzone die Vermischung des Sauerstoff enthaltenden Gasstromes mit dem kohlenwasserstoffhaltigen Einsatzstrom erfolgt und in den darauffolgend angeströmten Mischzonen jeweils eine Zwischenspeisung eines Sauerstoff enthaltenden Gasstromes in das noch zu dehydrierende, Kohlenwasserstoff enthaltende Reaktionsgemisch erfolgt.

Der Sauerstoff enthaltende Gasstrom wird über eine oder mehrere Zuführleitungen jeder der Mischzonen zugeführt, wobei jede Zuführleitung eine oder mehrere Verteilerkammern versorgt. Bei der Ausführungsform mit mehreren Zuführleitungen sind dieselben bevorzugt abhängig voneinander regelbar.

Der zu dehydrierende kohlenwasserstoffhaltige Gasstrom kann bevorzugt an zwei oder mehreren Stellen in den Wärmetauscher eingeleitet werden, insbesondere als ein Hauptstrom mit höherem Massenstrom und ein oder mehrere Nebenströme mit niedrigerem Massenstrom gegenüber dem Hauptstrom.

Für die Aufheizung des zu dehydrierenden kohlenwasserstoffhaltigen Gasstromes können zusätzlich zum Wärmetauscher eine oder mehrere Zusatzheizungen vorgesehen sein. Als Zusatzheizung kann bevorzugt die Zuführung von Wasserstoff durch die Zuführleitung für den zu dehydrierenden kohlenwasserstoffhaltigen Gasstrom möglichst nahe am Eintritt in die Mischzonen, die vor jeder katalytisch aktiven Zone angeordnet sind, vorgesehen sein.

Für die Aufheizung des Sauerstoff enthaltenden Gasstromes ist es alternativ auch möglich, dass Brennstoff, beispielsweise Wasserstoff oder Butan in eine oder sämtliche der Zuführleitungen für den Sauerstoff enthaltenden Gasstrom zudosiert wird, wobei die Konzentration des Brennstoffes im Sauerstoff enthaltenden Gasstrom dergestalt begrenzt werden muss, dass sie weit unterhalb der unteren Explosionsgrenze liegt. Vorteilhaft sollen die Konzentrationen des Brennstoffes in den Zuführleitungen für den Sauerstoff enthaltenden Gasstrom jeweils einzeln eingestellt werden können.

Gegenstand der Erfindung ist auch eine Anlage zur Durchführung einer autothermen Gasphasendehydrierung unter Verwendung eines vorstehend beschriebenen Reaktors, wobei für das aus dem Reaktor aus der Abführleitung austretende Reaktionsgasgemisch, bevorzugt nach einer Kondensation desselben, vor der Weiterleitung derselben zu einer Aufarbeitungsanlage ein Vorratsbehälter angeordnet ist.

Vorteilhaft wird der auch bereits nach dem Stand der Technik mit alternierendem Produktionsmodus/Regeneriermodus erforderliche Pufferbehälter gegenüber dem Stand der Technik doppelt oder dreifach so groß ausgelegt, um den geringen Zeitausfall durch die Regenerierphase zu kompensieren. Dadurch kann die Aufarbeitung nach der Dehydrierung weiterhin kontinuierlich laufen, wie gefordert.

Gegenstand der Erfindung ist auch die Verwendung des oben beschriebenen Reaktors oder einer oben beschriebenen Anlage in einem Verfahren zur Durchführung einer autothermen Gasphasendehydrierung.

Bevorzugt ist die autotherme Gasphasendehydrierung eine Dehydrierung von Propan, von Butan, von Isobutan, von Buten oder von Ethylbenzol.

Das erfindungsgemäße Verfahren ermöglicht es somit, eine autotherme Gasphasendehydrierung kontinuierlich in einem einzigen Reaktor, und entsprechend mit einer einzigen Katalysatorfüllung durchzuführen, ohne dass die Anlage zwecks Regenerierung des Katalysators abgestellt werden müsste. Dies entspricht einer Verfügbarkeit, gerechnet auf den einzelnen Reaktor, von 95 % gegenüber 50 % nach dem Stand der Technik. Beim alternativen Betrieb von zwei gleichartigen Reaktoren entfällt entsprechend auch die Umschaltproblematik, und die Peripherie ist entsprechend wesentlich einfacher. Darüber hinaus ist die Skalierbarkeit wesentlich kostengünstiger, da es im Gegensatz zum Stand der Technik nicht erforderlich ist, gleichzeitig zwei Reaktoren größer auszulegen. Darüber hinaus erhöht sich auch die Standzeit des Dehydrierkatalysators: aufgrund der erfindungsgemäßen Begrenzung der Betriebszeit für den Produktionsmodus können tiefgehende Ablagerungen an demselben vermieden werden, wodurch die Regenerierbarkeit des Katalysators verbessert wird.

Die Erfindung wird im Folgenden anhand eines Ausführungsbeispiels sowie von Zeichnungen näher erläutert.

### Ausführungsbeispiel

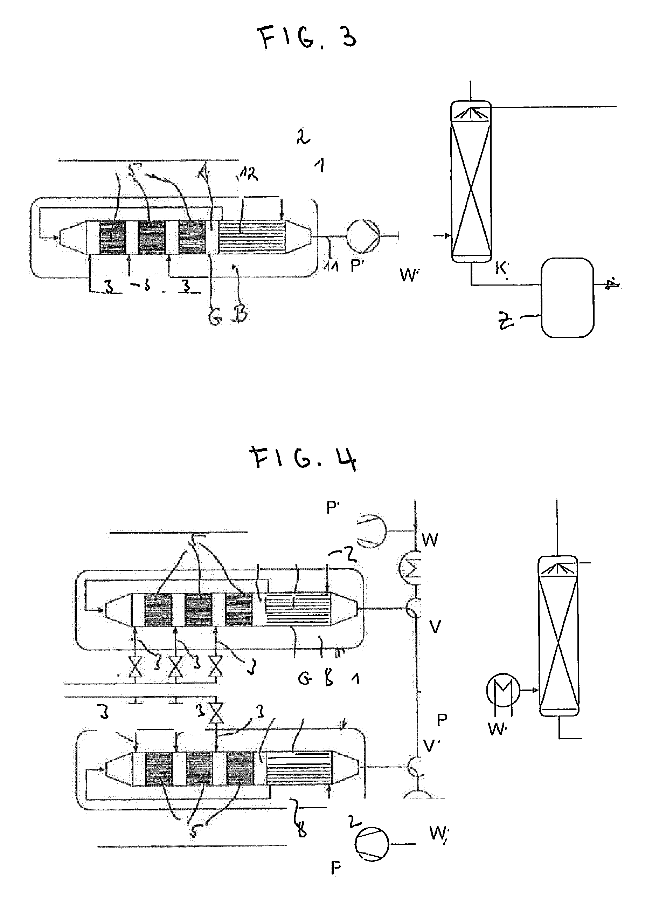

Eine Butan-Dehydrierung wurde zum einen in einer Anlage nach dem Stand der Technik, wie sie schematisch in Figur 4 dargestellt ist (Vergleichsbeispiel), zum anderen in einer Anlage mit einem einzigen Reaktor, wie sie schematisch in Figur 3 dargestellt ist (erfindungsgemäßes Beispiel), durchgeführt.

Dem Reaktor wurden 672,87 kg/h eines kohlenwasserstoffhaltigen Gasstromes mit einer Temperatur von 200 °C und einem Druck von 2 bar absolut mit der folgenden Zusammensetzung zugeführt:

| | **kg/h** |
|---|---|
| C₄H₁₀ | 507,3211 |
| C₄H₈ | 21,4382 |
| C₄H₆ | 1,3058 |
| H₂O | 133,3412 |
| CH₄ | 4,1280 |
| COₓ | 0,0000 |
| O₂ | 0,0000 |
| H₂ | 5,3395 |
| N₂ | 0,0000 |

Weiterhin wurde dem Reaktor ein Sauerstoff enthaltender Gasstrom 3 mit einer Temperatur von 240 °C und einem Druck von 2 bar absolut über drei unabhängig voneinander regelbare Leitungen 9 zugeführt, wobei der in Strömungsrichtung ersten angeordneten Zuführleitung ein Gasstrom von 20,32 kg/h Sauerstoff und 69,44 kg/h Wasserdampf, der in Strömungsrichtung durch den Reaktor an zweiter Stelle angeordneten Zuführleitung 9 ein Gasstrom von 11,45 kg/h Sauerstoff und 39,12 kg/h Wasserdampf und der in Strömungsrichtung an dritter Stelle angeordneten Zuführleitung 9 ein 8,57 kg/h Sauerstoff und 29,29 kg/h Wasserdampf enthaltender Gasstrom 3 zugeführt wurde.

### Vergleichsbeispiel:

Bei der Durchführung der autothermen Gasphasendehydrierung unter den obigen Betriebsbedingungen in einer Anlage entsprechend Figur 4 über eine gesamte Betriebszeit von 12 h wurde der erste Reaktor im Produktionsmodus und der zweite Reaktor im Regeneriermodus betrieben. Der Umsatz betrug zum Startzeitpunkt 40,5 % und zum Ende der Betriebszeit von 12 h nur 39,65 %, d.h. im zeitlichen Mittelwert 40,00 %.

### Beispiel (erfindungsgemäß.):

Unter denselben Betriebsbedingungen wurde eine Anlage entsprechend der schematischen Darstellung in Figur 3, d.h. mit einem einzigen Reaktor, betrieben. Die Betriebszeit für den Produktionsmodus betrug 3 Stunden, und die Betriebszeit für den Regeneriermodus 10 min, d.h. lediglich etwa 5 % der Gesamtzeit eines Betriebszyklus. Der Umsatz betrug während der gesamten Betriebszeit für den Produktionsmodus ca. 40,5 %.

In der Zeichnung zeigen im Einzelnen:
- Figur 1A: eine schematische Darstellung der bevorzugten Ausführungsform eines erfindungsgemäßen Reaktors 1 mit beispielhaft drei hintereinander angeordneten katalytisch aktiven Zonen 5 und einem Wärmetauscher 12, wobei T1 bis T3 als die Temperaturen am Austritt aus der ersten, zweiten bzw. dritten katalytisch aktiven Zone 5 bezeichnet werden,

- Figur 1C: eine schematische Darstellung der Änderung des Umsatzes in Prozenten, in Abhängigkeit von der Zeit in Sekunden,
- Figur 2A: einen Längsschnitt durch eine bevorzugte Ausführungsform eines erfindungsgemäßen Reaktors in horizontaler Ebene,
- Figur 2B: einen Längsschnitt durch denselben Reaktor in vertikaler Ebene,
- Figur 3: eine schematische Darstellung in einer bevorzugten Ausführungsform für eine Anlage zur Durchführung einer autothermen Gasphasendehydrierung nach dem erfindungsgemäßen Verfahren und
- Figur 4: eine schematische Darstellung einer Anlage zur Durchführung einer autothermen Gasphasendehydrierung nach dem Stand der Technik.

In den Figuren bezeichnen gleiche Bezugszeichen jeweils gleiche oder entsprechende Merkmale.

Die schematische Darstellung eines bevorzugten erfindungsgemäßen Reaktors mit drei hintereinander angeordneten katalytisch aktiven Zonen 5 verdeutlicht die Stellen im Reaktor, an denen die Temperaturen T1 bis T3 gemessen werden, d.h. Temperatur T1 beim Austritt aus der festem, vom Reaktionsgemisch durchströmten katalytisch aktiven Zone 5, die Temperatur T2 beim Austritt aus der in Strömungsrichtung zweiten, vom Reaktionsgasgemisch durchströmten katalytisch aktiven Zone und Temperatur T3 beim Austritt aus der dritten letzten, in Strömungsrichtung angeordneten katalytisch aktiven Zone 5 und vor dem Eintritt in den Wärmetauscher 12.

Die Temperatur des Reaktionsgasgemisches beim Austritt desselben aus der Abführleitung in der Startphase des Produktionsmodus zunächst schwankt, bis sich ein stationärer Zustand einstellt, ab dem die Temperaturerhöhung weitgehend linear ist. Der Zeitpunkt, ab dem sich der stationäre Zustand einstellt, wird als Zeitpunkt Null (t_{ref}) (Startpunkt) für die Bestimmung der Temperaturerhöhung, ab der der Produktionsmodus abgebrochen wird und der Reaktor in den Regeneriermodus geschaltet wird, festgelegt.

Der Zeitpunkt Null (t_{ref}) wird festgelegt als der Zeitpunkt ab dem die Temperatur T2 gegenüber der Temperatur T1 steiler ansteigt und ebenso die Temperatur T3 gegenüber der Temperatur T2.

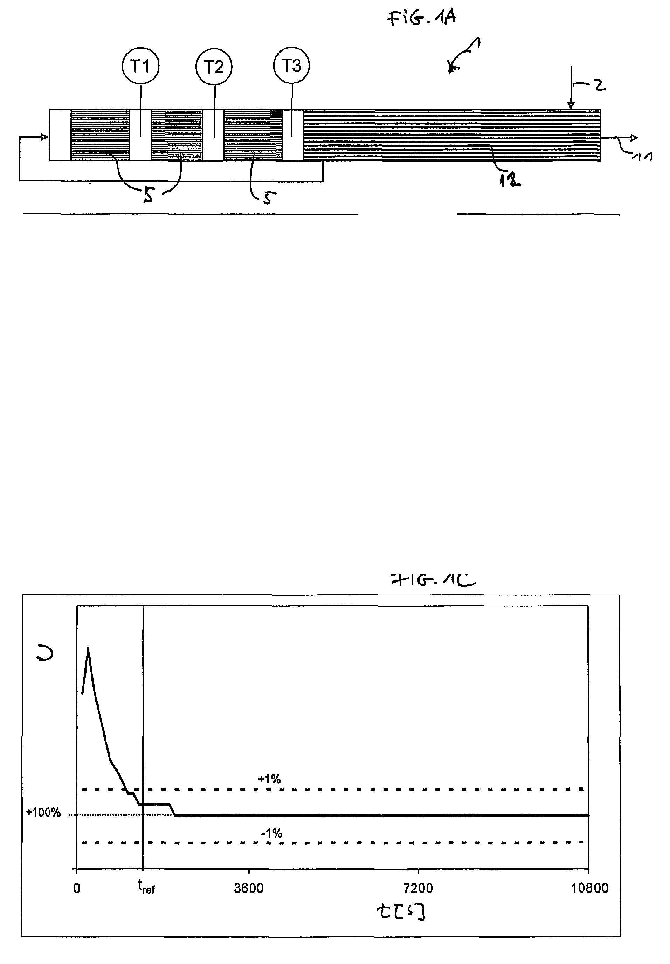

Figur 1C verdeutlicht entsprechende Änderungen des normierten Umsatzes U in Prozenten gegenüber der Zeit in Sekunden: Zeitpunkt Null (t_{ref}) entspricht dem Zeitpunkt, ab dem der Umsatz, wie er schematisch in Figur 1C dargestellt ist, in einen stationären Zustand übergeht, d.h. um nicht mehr als 1 %, bezogen auf den Endumsatz, bevorzugt um nicht mehr als 0,5 %, bezogen auf den Endumsatz, weiter bevorzugt um nicht mehr als 0,2 %, bezogen auf den Endumsatz, schwankt.

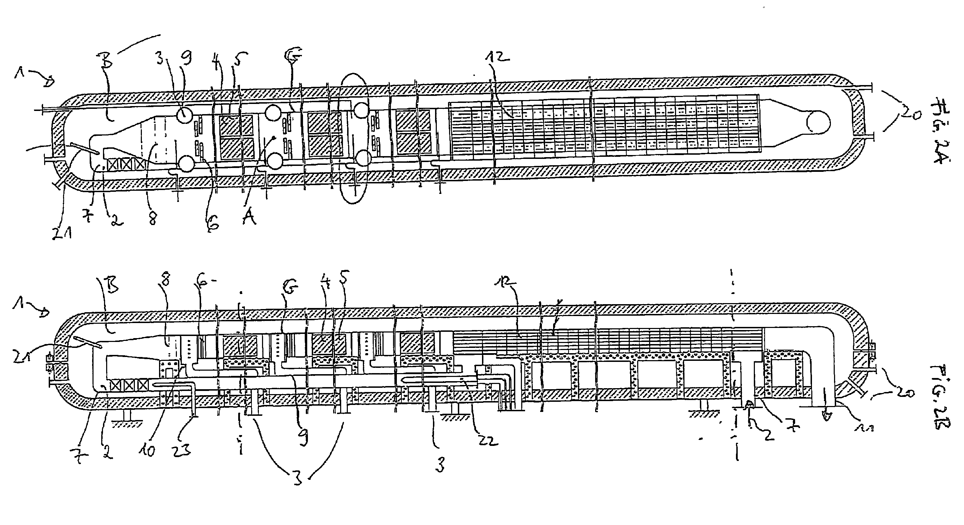

Die Längsschnittdarstellung in horizontaler Ebene in Figur 2A zeigt schematisch eine bevorzugte Ausführungsform eines Reaktors zur Durchführung des erfindungsgemäßen Verfahrens. Das Gehäuse G teilt den Innenraum des Reaktors in einen Innenbereich A und einen Außenbereich B. Der Reaktor wird mit dem zu dehydrierenden kohlenwasserstoffhaltigen Gasstrom 2 über eine Zuführleitung 7 und drei Zuführleitungen 9 mit einem Sauerstoff enthaltenden Gasstrom 3 gespeist. Im Innenbereich A des Reaktors sind beispielhaft drei katalytisch aktive Zonen 5 angeordnet, die aus Monolithen 4 gebildet sind. In jeder katalytisch aktiven Zone 5 ist jeweils Zone 6 mit festen Einbauten angeordnet. Der wasserstoffhaltige Gasstrom 2 wird über einen Wärmetauscher 12 in indirektem Wärmetausch mit dem Reaktionsgasgemisch aufgeheizt, in das andere Ende des Reaktors geleitet, dort umgelenkt und über einen Strömungsgleichrichter 8 in den Innenbereich A eingeleitet, in dem in den katalytisch aktiven Zonen 5 die autotherme Gasphasendehydrierung stattfindet. Die bevorzugte in Figur 2A dargestellte Ausführungsform zeigt auf der rechten Seite der Darstellung Zuführleitungen 20 für einen Purge-Gasstrom und auf der linken Seite der Darstellung eine Verbindungsleitung 21 für den Purge-Gasstrom aus dem Außenbereich B des Reaktors sowie die Zuführleitung 7 für den zu dehydrierenden kohlenwasserstoffhaltigen Gasstrom 2.

Die Längsdarstellung in vertikaler Ebene in Figur 2B verdeutlicht darüber hinaus bevorzugte Einrichtungen, und zwar Zusatzheizungen, die vorteilhaft eingesetzt werden können: eine elektrische Heizung 22 sowie eine Zuführleitung 23 für Wasserstoff als Brenngas in die Zuführleitung 7 für den zu dehydrierenden kohlenwasserstoffhaltigen Gasstrom 2.

Figur 3 zeigt eine schematische Darstellung einer bevorzugten Anlage zur Durchführung des erfindungsgemäßen Verfahrens in einem einzigen Reaktor 1 mit Gehäuse G, das den Reaktorinnenraum in einen Innenbereich A und einen Außenbereich B aufteilt, wobei im Innenbereich A beispielhaft drei katalytisch aktive Zonen 5 aus nicht im Einzelnen dargestellten Monolithen angeordnet sind, sowie einen Wärmetauscher 12. Dem Reaktor 1 wird ein kohlenwasserstoffhaltiger Gasstrom 2 sowie ein Sauerstoff enthaltender Gasstrom 3 in drei Teilströmen zugeführt. Das Reaktionsgasgemisch wird über die Abführleitung 11 aus dem Reaktor abgezogen, über eine Pumpe P und einen Wärmetauscher W einer Waschkolonne K, und weiter einem Zwischenpuffer Z zugeführt, vor der Weiterleitung zur insbesondere destillativen Aufarbeitung.

Die schematische Darstellung in Figur 4 zeigt demgegenüber eine Anlage nach dem Stand der Technik, aufweisend zwei gleichartige Reaktoren 1.

## Patentansprüche

1. Verfahren zur autothermen Gasphasendehydrierung eines kohlenwasserstoffhaltigen Gasstromes (2) mit einem Sauerstoff enthaltenden Gasstrom (3) unter Erhalt eines Reaktionsgasgemisches an einem heterogenen Katalysator, der als Monolith (4) ausgebildet ist, und Regeneration des Katalysators in einem Reaktor (1) in Form eines Zylinders oder Prismas, wobei
- der Innenraum des Reaktors (1) durch ein in Längsrichtung des Reaktors (1) angeordnetes kreiszylindrisches oder prismatisches gasdichtes Gehäuse G in
- einen Innenbereich A, mit einer oder mehreren hintereinander angeordneten katalytisch aktiven Zonen (5), worin jeweils eine Packung aus aufeinander, nebeneinander und hintereinander gestapelten Monolithen (4) und vor jeder katalytisch aktiven Zone (5) jeweils eine Mischzone (6) mit festen Einbauten vorgesehen ist und
- einen koaxial zum Innenbereich A angeordneten Außenbereich B aufgeteilt ist, und wobei
- an einem Reaktorende im Anschluss an das Gehäuse G ein Wärmetauscher (12) vorgesehen ist
- mit einer oder mehreren Zuführleitungen (7) für den zu dehydrierenden kohlenwasserstoffhaltigen Gasstrom (2),
- mit einer oder mehreren Zuführleitungen (9) für den Sauerstoff enthaltenden Gasstrom (3) in jeder der Mischzonen (6), wobei jede Zuführleitung (9) eine oder mehrere Verteilerkammern (10) versorgt, sowie
- mit einer Abführleitung (11) für das Reaktionsgasgemisch der autothermen Gasphasendehydrierung, wobei
- der Außenbereich B mit einem unter den Reaktionsbedingungen der autothermen Gasphasendehydrierung inerten Gas beaufschlagt ist und
- der zu dehydrierende kohlenwasserstoffhaltige Gasstrom (2) über eine Zuführleitung (7) in den Wärmetauscher (12) eingeleitet, im Wärmetauscher (12) durch das Reaktionsgasgemisch im Gegenstrom durch indirekten Wärmetausch aufgeheizt und weiter an das dem Wärmetauscher (12) entgegengesetzte Ende des Reaktors geleitet, dort umgelenkt, über einen Strömungsgleichrichter (8) in den Innenbereich A eingeleitet, und in den Mischzonen (6) mit dem Sauerstoff enthaltenden Gasstrom (3) vermischt wird, worauf im Innenbereich A des Reaktors (1) die autotherme Gasphasendehydrierung stattfindet, **dadurch gekennzeichnet, dass**
- der Reaktor alternierend im Produktionsmodus der autothermen Gasphasendehydrierung und im Regeneriermodus betrieben wird, wobei
- der Produktionsmodus der autothermen Gasphasendehydrierung solange gefahren wird, solange die Temperaturerhöhung des Reaktionsgasgemisches nach dem Austritt aus der letzten in Strömungsrichtung angeordneten katalytisch aktiven Zone (5) und vor dem Eintritt in den Wärmetauscher (12), bezogen auf den Zeitpunkt, ab dem
- bei Einsatz einer einzigen katalytisch aktiven Zone (5) die Temperaturerhöhung des Reaktionsgasgemisches nach dem Austritt aus der einzigen katalytisch aktiven Zone (5) und vor dem Eintritt in den Wärmetauscher (12) über einen Zeitraum von mindestens 15 Minuten linear ansteigt, oder
- bei Einsatz von zwei oder mehreren nacheinander angeordneten katalytisch aktiven Zonen (5) die Temperaturerhöhung des Reaktionsgasgemisches beim Austritt aus jeder katalytisch aktiven Zone (5) gegenüber der Temperaturerhöhung beim Austritt aus der unmittelbar vorhergehenden katalytisch aktiven Zone (5) stärker ansteigt,
5 K nicht überschreitet, worauf
- der Reaktor in den Regeneriermodus unter Zuführung eines inerten Regeneriergases geschaltet wird, das mindestens 10 Gew.-% Sauerstoff, bezogen auf das Gesamtgewicht des Regeneriergases, enthält.

2. Verfahren nach Anspruche 1, **dadurch gekennzeichnet, dass** der Produktionsmodus der autothermen Gasphasendehydrierung beendet und der Reaktor (1) in den Regeneriermodus umgeschaltet wird, sobald die Temperaturerhöhung des Reaktionsgasgemisches beim Austritt aus der letzten katalytisch aktiven Zone (5) und vor dem Eintritt in den Wärmetauscher (12), bezogen auf den in Anspruch 1 definierten Zeitpunkt, 4 K überschreitet.

3. Verfahren nach Anspruch 1, **dadurch gekennzeichnet, dass** der Produktionsmodus der autothermen Gasphasendehydrierung beendet und der Reaktor (1) in den Regeneriermodus umgeschaltet wird, sobald die Temperaturerhöhung des Reaktionsgasgemisches beim Austritt aus der letzten katalytisch aktiven Zone (5) und vor dem Eintritt in den Wärmetauscher (12), bezogen auf den in Anspruch 1 definierten Zeitpunkt, 3 K überschreitet.

4. Verfahren nach einem der Ansprüche 1 bis 3, **dadurch gekennzeichnet, dass** in jedem Betriebszyklus, umfassend jeweils einen Produktionsmodus und einen Regeneriermodus, maximal 15 % der gesamten Betriebszeit auf den Regeneriermodus entfällt.

5. Verfahren nach Anspruch 4, **dadurch gekennzeichnet, dass** in jedem Betriebszyklus, umfassend jeweils einen Produktionsmodus und einen Regeneriermodus, maximal 10 %, bevorzugt maximal 5 %, der gesamten Betriebszeit auf den Regeneriermodus entfällt.

6. Verfahren nach einem der Ansprüche 1 bis 5, **dadurch gekennzeichnet, dass** der zu dehydrierende kohlenwasserstoffhaltige Gasstrom (2), an zwei oder mehreren Stellen in den Wärmetauscher (12) eingeleitet wird, bevorzugt als ein Hauptstrom mit höherem Massenstrom und als ein oder mehrere Nebenströme mit niedrigerem Massenstrom gegenüber dem Hauptstrom.

7. Verfahren nach einem der Ansprüche 1 bis 6, **dadurch gekennzeichnet, dass** zusätzlich zum Wärmetauscher (12) eine oder mehrere Zusatzheizungen für den zu dehydrierenden kohlenwasserstoffhaltigen Gasstrom (2) vorgesehen sind.

8. Verfahren nach einem der Ansprüche 1 bis 7, **dadurch gekennzeichnet, dass** im Innenbereich A zwei oder mehrere katalytisch aktive Zonen (5) mit jeweils einer Packung aus aufeinander, nebeneinander und hintereinander gestapelten Monolithen (4) vorgesehen sind,
- wobei die Monolithe (4) innerhalb derselben katalytisch aktiven Zone (5), bevorzugt mit jeweils unterschiedlicher katalytischer Aktivität ausgebildet sind, und/oder
- wobei die zwei oder mehreren katalytisch aktiven Zonen (5) mit jeweils unterschiedlicher katalytischer Aktivität ausgebildet sind.

9. Verfahren nach einem der Ansprüche 1 bis 8, **dadurch gekennzeichnet**, das Gehäuse G als Prisma ausgebildet ist, und dass die Seitenwände des als Prisma ausgebildeten Gehäuses G einzeln abnehmbar ausgebildet sind, dergestalt, dass eine komplette Packung oder einzelne Monolithe (4) einer Packung aus einer katalytisch aktiven Zone (5) ausgetauscht werden können.

10. Verfahren nach einem der Ansprüche 1 bis 9, **dadurch gekennzeichnet, dass** für das aus dem Reaktor (1) aus der Abführleitung (11) austretende Reaktionsgasgemisch, bevorzugt nach einer Kondensation desselben und vor der Weiterleitung desselben zu einer Aufarbeitungsanlage, ein Vorratsbehälter vorgesehen ist.

11. Verfahren nach einem der Ansprüche 1 bis 10, **dadurch gekennzeichnet, dass** die autotherme Gasphasendehydrierung eine Dehydrierung von Propan, von Butan, von Isobutan, von Buten oder von Ethylbenzol ist.

## Claims

1. A process for the autothermal gas-phase dehydrogenation of a hydrocarbon-comprising gas stream (2) by means of an oxygen-comprising gas stream (3) over a heterogeneous catalyst configured as a monolith (4) to give a reaction gas mixture and regeneration of the catalyst in a reactor (1) in the form of a cylinder or prism, where
- the interior space of the reactor (1) is divided by means of a cylindrical or prismatic gastight housing G arranged in the longitudinal direction of the reactor (1) into
- an inner region A which has one or more catalytically active zones (5) arranged after one another and in which a packing composed of monoliths (4) stacked on top of one another, next to one another and after one another is provided in each catalytically active zone (5) and a mixing zone (6) having fixed internals is provided before each catalytically active zone (5) and
- an outer region B arranged coaxially with the inner region A, and
- a heat exchanger (12) is provided at one end of the reactor next to the housing G,
- with one or more feed lines (7) for the hydrocarbon-comprising gas stream (2) to be dehydrogenated,
- with one or more feed lines (9) for the oxygen-comprising gas stream (3) in each of the mixing zones (6), where each feed line (9) supplies one or more distributor chambers (10), and
- with a discharge line (11) for the reaction gas mixture of the autothermal gas-phase dehydrogenation, where
- the outer region B is supplied with a gas which is inert under the reaction conditions of the autothermal gas-phase dehydrogenation and
- the hydrocarbon-comprising gas stream (2) to be dehydrogenated is introduced via a feed line (7) into the heat exchanger (12), heated in the heat exchanger (12) by indirect heat exchange in countercurrent with the reaction gas mixture and conveyed further to the end of the reactor opposite the heat exchanger (12), diverted there, introduced via a flow equalizer (8) into the inner region A and mixed in the mixing zones (6) with the oxygen-comprising gas stream (3), whereupon the autothermal gas-phase dehydrogenation takes place in the inner region A of the reactor (1), wherein
- the reactor is operated alternately in the production mode of the autothermal gas-phase dehydrogenation and in the regeneration mode, where
- the production mode of the autothermal gas-phase dehydrogenation is operated as long as the increase in temperature of the reaction gas mixture after exit from the last catalytically active zone (5) viewed in the flow direction and before entry into the heat exchanger (12), based on the point in time after which
- when using a single catalytically active zone (5), the increase in temperature of the reaction gas mixture increases linearly after exit from the single catalytically active zone (5) and before entry into the heat exchanger (12) over a period of at least 15 minutes, or
- when using two or more catalytically active zones (5) arranged after one another, the increase in temperature of the reaction gas mixture increases to a greater extent on exit from each catalytically active zone (5) compared to the increase in temperature on exit from the immediately preceding catalytically active zone (5),
does not exceed 5 K, whereupon
- the reactor is switched over to the regeneration mode with introduction of an inert regeneration gas which comprises at least 10% by weight of oxygen, based on the total weight of the regeneration gas.

2. The process according to claim 1, wherein the production mode of the autothermal gas-phase dehydrogenation is ended and the reactor (1) is switched over to the regeneration mode as soon as the increase in temperature of the reaction gas mixture on exiting the last catalytically active zone (5) and before entry into the heat exchanger (12), based on the point in time defined in claim 1, exceeds 4 K.

3. The process according to claim 1, wherein the production mode of the autothermal gas-phase dehydrogenation is ended and the reactor (1) is switched over to the regeneration mode as soon as the increase in temperature of the reaction gas mixture on exiting the last catalytically active zone (5) and before entry into the heat exchanger (12), based on the point in time defined in claim 1, exceeds 3 K.

4. The process according to any of claims 1 to 3, wherein not more than 15% of the total operating time is taken up by the regeneration mode in each operating cycle comprising in each case one production mode and one regeneration mode.

5. The process according to claim 4, wherein not more than 10%, preferably not more than 5%, of the total operating time is taken up by the regeneration mode in each operating cycle comprising in each case one production mode and one regeneration mode.

6. The process according to any of claims 1 to 5, wherein the hydrocarbon-comprising gas stream (2) to be dehydrogenated is introduced into the heat exchanger (12) at two or more places, preferably as a main stream having a higher mass flow and one or more secondary streams having a lower mass flow than the main stream.

7. The process according to any of claims 1 to 6, wherein one or more additional heating means in addition to the heat exchanger (12) are provided for the hydrocarbon-comprising gas stream (2) to be dehydrogenated.

8. The process according to any of claims 1 to 7, wherein two or more catalytically active zones (5) each having a packing composed of monoliths (4) stacked on top of one another, next to one another and behind one another are provided in the inner region A, where
- the monoliths (4) within the same catalytically active zone (5) preferably each have a different catalytic activity and/or
- the two or more catalytically active zones (5) each have a different catalytic activity.

9. The process according to any of claims 1 to 8, wherein the housing G is configured as a prism and the side walls of the housing G configured as a prism are configured so as to be able to be taken off individually, so that a complete packing or individual monoliths (4) of a packing of a catalytically active zone (5) can be replaced.

10. The process according to any of claims 1 to 9, wherein a reservoir is provided for the reaction gas mixture leaving the reactor (1) via the discharge line (11), preferably after condensation of the reaction gas mixture and before the reaction gas mixture is passed onto a work-up plant.

11. The process according to any of claims 1 to 10, wherein the autothermal gas-phase dehydrogenation is a dehydrogenation of propane, of butane, of isobutane, of butene or of ethylbenzene.

## Revendications

1. Procédé de déshydrogénation autotherme en phase gazeuse d'un écoulement (2) de gaz contenant du carbone à l'aide d'un écoulement (3) de gaz contenant de l'oxygène avec obtention d'un mélange gazeux de réaction sur un catalyseur hétérogène configuré comme monolithe (4) et régénération du catalyseur dans un réacteur (1) qui présente la forme d'un cylindre ou d'un prisme,
l'espace intérieur du réacteur (1) étant divisé par un boîtier G cylindrique circulaire ou prismatique, étanche aux gaz, disposé dans le sens de la longueur du réacteur (1) en
une partie intérieure A qui présente une ou plusieurs zones (5) catalytiquement actives disposées les unes à la suite des autres, une garniture constituée de monolithes (4) empilés les uns sur les autres, les uns à côté des autres et les uns derrière les autres et une zone de mélange (6) dotée de garnitures solides en amont de chaque zone (5) catalytiquement active et
une partie extérieure B disposée coaxialement par rapport à la partie intérieure A,
un échangeur de chaleur (12) étant prévu à une extrémité du réacteur et se raccordant au boîtier G,
présentant un ou plusieurs conduits d'amenée (7) pour l'écoulement (2) de gaz contenant du carbone et à déshydrogéner,
un ou plusieurs conduits d'amenée (9) de l'écoulement (3) de gaz contenant de l'oxygène dans chacune des zones de mélange (6), chaque conduit d'amenée (9) alimentant une ou plusieurs chambres de répartition (10) et
un conduit d'évacuation (11) du mélange de gaz de réaction de la déshydrogénation autotherme en phase gazeuse,
la partie extérieure B étant alimentée en un gaz qui est inerte dans les conditions de réaction de la déshydrogénation autotherme en phase gazeuse et
l'écoulement (2) de gaz contenant du carbone et à déshydrogéner étant introduit par un conduit d'amenée (7) dans l'échangeur de chaleur (12), étant chauffé à contre-courant dans l'échangeur de chaleur (12) par échange de chaleur indirect avec le mélange de gaz de réaction et étant amené à l'extrémité du réacteur opposée à l'échangeur de chaleur (12) pour y être dévié, être introduit dans la partie intérieure A par un redresseur d'écoulement (8) et mélangé dans les zones de mélange (6) avec l'écoulement (3) de gaz contenant de l'oxygène, suite à quoi la déshydrogénation autotherme en phase gazeuse a lieu dans la partie intérieure A du réacteur (1), **caractérisé en ce que**
le réacteur est conduit en alternance en mode de production de déshydrogénation autotherme en phase gazeuse et en mode de régénération,
**en ce que** le mode de production de déshydrogénation autotherme en phase gazeuse est maintenu tant que l'augmentation de température du mélange de réaction après la sortie hors de la dernière zone (5) catalytiquement active dans la direction d'écoulement et en amont de l'entrée dans l'échangeur de chaleur (12) ne dépasse pas 5 K à partir de l'instant auquel la température du mélange de gaz de réaction après la sortie hors de la zone (5) catalytiquement active unique et avant l'entrée dans l'échangeur de chaleur (12) augmente linéairement en une durée d'au moins 15 minutes lorsque l'on utilise une seule zone (5) catalytiquement active, ou
à partir de l'instant auquel la température du mélange de gaz de réaction à la sortie de chaque zone (5) catalytiquement active par rapport à l'augmentation de température lors de la sortie hors de la zone (5) catalytiquement active immédiatement précédente augmente fortement lorsque l'on utilise deux ou plusieurs zones (5) catalytiquement actives disposées les unes à la suite des autres,
suite à quoi le réacteur est commuté en mode de régénération par amenée d'un gaz inerte de régénération qui contient au moins 10 % en poids d'oxygène par rapport au poids total du gaz de régénération.

2. Procédé selon la revendication 1, **caractérisé en ce que** le mode de production de la déshydrogénation autotherme en phase gazeuse se termine et le réacteur (1) est commuté en mode de régénération dès que l'augmentation de température du mélange de réaction à la sortie hors de la dernière zone (5) catalytiquement active et avant l'entrée dans l'échangeur de chaleur (12) dépasse 4 K par rapport à l'instant défini en revendication 1.

3. Procédé selon la revendication 1, **caractérisé en ce que** le mode de production de la déshydrogénation autotherme en phase gazeuse se termine et le réacteur (1) est commuté en mode de régénération dès que l'augmentation de température du mélange de gaz de réaction à la sortie hors de la dernière zone (5) catalytiquement active et avant l'entrée dans l'échangeur de chaleur (12) dépasse 3 K par rapport à l'instant défini à la revendication 1.

4. Procédé selon l'une des revendications 1 à 3, **caractérisé en ce qu'**en chaque cycle de fonctionnement qui comprend un mode de production et un mode de régénération, au plus 15 % de l'ensemble de la durée de fonctionnement se trouvent en mode de régénération.

5. Procédé selon la revendication 4, **caractérisé en ce que** dans chaque cycle de fonctionnement qui comprend un mode de production et un mode de régénération, au plus 10 % et de préférence au plus 5 % de l'ensemble de la durée de fonctionnement se trouvent en mode de régénération.

6. Procédé selon l'une des revendications 1 à 5, **caractérisé en ce que** l'écoulement (2) de gaz contenant du carbone et à déshydrogéner est introduit en deux ou plusieurs emplacements dans l'échangeur de chaleur (12), de préférence sous la forme d'un écoulement principal présentant un écoulement massique plus élevé ou sous la forme d'un ou plusieurs écoulements secondaires dont l'écoulement massique est plus bas que l'écoulement principal.

7. Procédé selon l'une des revendications 1 à 6, **caractérisé en ce qu'**en plus de l'échangeur de chaleur (12), un ou plusieurs chauffages supplémentaires sont prévus pour l'écoulement (2) de gaz contenant du carbone et à déshydrogéner.

8. Procédé selon l'une des revendications 1 à 7, **caractérisé en ce que** deux ou plusieurs zones (5) catalytiquement actives qui présentent chacune une garniture constituée de plusieurs monolithes (4) empilés les uns sur les autres, les uns à côté des autres et les uns derrière les autres sont prévues dans la partie intérieure A,
les monolithes (4) prévus à l'intérieur d'une même zone (5) catalytiquement active présentant de préférence des activités catalytiques différentes, et/ou
les deux ou plusieurs zones (5) catalytiquement actives présentant chacune une activité catalytique différente.

9. Procédé selon l'une des revendications 1 à 8, **caractérisé en ce que** le boîtier G est configuré en prisme et **en ce que** les parois latérales du boîtier G configuré en prisme peuvent être enlevées séparément de telle sorte que l'on puisse remplacer l'ensemble de la garniture ou certains monolithes (4) d'une garniture d'une zone (5) catalytiquement active.

10. Procédé selon l'une des revendications 1 à 9, **caractérisé en ce qu'**un récipient de réserve est prévu pour le mélange de gaz de réaction qui sort du réacteur (1) par le conduit d'évacuation (11), de préférence après sa condensation et avant son envoi dans une installation de traitement.

11. Procédé selon l'une des revendications 1 à 10, **caractérisé en ce que** la déshydrogénation autotherme en phase gazeuse est une déshydrogénation de propane, de butane, d'isobutane, de butène ou d'éthylbenzène.
